# EUROPEAN PATENT SPECIFICATION

(11) **EP 2 739 730 B1**
(45) Date of publication and mention of the grant of the patent: **15.05.2019**
(21) Application number: 12820186.0
(22) Date of filing: 03.08.2012
(51) Int. Cl.: C12N 15/09, C12N 15/87, C07K 14/005, C12N 15/33, C07K 14/195, C12N 15/31

(54) **CELL FREE TRANSLATION SYSTEM FOR COMPOUND SCREENING AND RELATED USES**
ZELLFREIES TRANSLATIONSSYSTEM ZUM SCREENING VON VERBINDUNGEN UND VERWANDTE VERWENDUNGEN
SYSTÈME DE TRANSLATION ACELLULAIRE POUR CRIBLAGE DE COMPOSÉ ET UTILISATIONS AFFÉRENTES

(30) Priority: 03.08.2011 US 201161514825 P
(43) Date of publication of application: 11.06.2014
(73) Proprietor: Prosetta Antiviral Inc., San Francisco, California 94107 (US)
(72) Inventor: LINGAPPA, Vishwanath, R., San Francisco, CA 94116 (US)
(74) Representative: Harrison IP Limited
(86) International application number: PCT/US2012/049625
(87) International publication number: WO 2013/020101

(56) References cited:
- EP-A1- 1 314 781
- EP-A1- 1 316 617
- KR-A- 20030 031 992
- US-A1- 2003 104 577
- US-A1- 2006 177 813
- US-A1- 2007 128 633
- MOLLA A ET AL: "CELL-FREE DE-NOVO SYNTHESIS OF POLIOVIRUS", SCIENCE (WASHINGTON D C), vol. 254, no. 5038, 1991, pages 1647-1651, XP55166884, ISSN: 0036-8075
- LINGAPPA V R ET AL: "Cell-Free Protein Synthesizing Systems As Tools for Discovery of Drugs Inhibiting Viral Capsid Assembly", ANTIVIRAL RESEARCH, ELSEVIER BV, NL, vol. 86, no. 1, 1 April 2010 (2010-04-01), page A61, XP027018360, ISSN: 0166-3542 [retrieved on 2010-04-01]
- LINGAPPA V R ET AL: "Small Molecule Inhibitors of De Novo Cell-free Capsid Assembly Effective against Flaviviridae and Togaviridae", ANTIVIRAL RESEARCH, ELSEVIER BV, NL, vol. 86, no. 1, 1 April 2010 (2010-04-01), page A37, XP027018292, ISSN: 0166-3542 [retrieved on 2010-04-01]
- LINGAPPA, JR. ET AL.: 'A Eukaryotic Cytosolic Chaperonin Is Associated with a High Molecular Weight Intermediate in the Assembly of Hepatitis B Virus Ca psid, a Multimeric Particle' THE JOURNAL OF CELL BIOLOGY vol. 125, no. 1, April 1994, pages 99 - 111, XP055143536
- BRUENING G ET AL: "In vitro and in vivo translation of the ribonucleic acids of a cowpea strain of tobacco mosaic virus", VIROLOGY, ELSEVIER, AMSTERDAM, NL, vol. 71, no. 2, 1 June 1976 (1976-06-01), pages 498-517, XP023054662, ISSN: 0042-6822, DOI: 10.1016/0042-6822(76)90377-9 [retrieved on 1976-06-01]

## Description

### CROSS-REFERENCES TO RELATED APPLICATIONS

The present application claims the benefit of U.S. Provisional Application No. 61/514,825, filed August 3, 2011.

### STATEMENT AS TO RIGHTS TO INVENTIONS MADE UNDER FEDERALLY SPONSORED RESEARCH AND DEVELOPMENT

Not applicable

### FIELD OF THE INVENTION

The present invention relates to a wheat germ extract for use in compositions for cell-free translation, methods of expressing proteins using a composition including wheat germ extract, methods of assembling viral capsids, and methods of detecting compounds that modulate protein-protein interactions including but not limited to the assembly of viral capsids.

### BACKGROUND OF THE INVENTION

Cell-free translation systems utilize cellular extracts to express proteins of interest. In this paradigm, an in vitro transcribed or isolated RNA template is added to the cellular extract along with additional components, i.e. amino acids, to allow protein synthesis.

Wheat germ extract is commonly used for ceil-free translation reactions, and was initially described by various investigators (e.g., Roberts, B. E. and Paterson, B. M. (1973, PNAS 70, p. 2330)). Wheat germ extract is desirable because is supports translation of prokaryotic, eukaryotic, and viral RNAs. Wheat germ extract has been further shown to be useful in cell-free systems designed to assemble viral capsids (Lingappa et al. J Cell Bio 125: 99-111 (1994); Lingappa et al. J Cell Bio 136:567-581 (1997); Singh et al. Virology 279:257-270 (2001); Zimmerman et al. Nature 415:88-92 (2002); Lingappa and Thielen, Methods Mol Biol. 485:185-95 (2009)). Viral capsids are the protein shell of a virus that protects the viral genome, and its assembly is a process, catalyzed by host factors, that can be targeted to develop anti-viral drugs.

Proper wheat germ extract concentration is required for efficient protein expression. Optimal wheat germ extract concentration for robust protein expression has generally been shown to be around 40 to 50% of the total translation mix (Erikson and Blobel, Methods Enzymology 96:38-50 (1983)). Reports have specified that wheat germ extract is optimally used at 20% of the final reaction volume for proper capsid assembly (Lingappa and Thielen, Methods Mol Biol. 485: 185-95 (2009)).

It is an important aspect of antiviral drug development processes utilizing cell-free expression systems to show that antiviral modulator analogs are effective in cell-free systems in similar relative proportion to their activity against viruses in cell culture. Currently used cell-free systems do not show useful correlation between the ability of therapeutic agents that disrupt capsid assembly *in vitro* and the ability of these therapeutic agents to prevent virus replication. There is therefore a need to develop a cell-free system having levels and ratios of components that achieve maximum sensitivity to drugs in the cell-free system and then to correlate that efficacy against live viruses in cell culture.

### BRIEF SUMMARY OF THE INVENTION

Quite surprisingly, it has now been discovered that cell-free systems in which the concentration of wheat germ extract is maintained at or below about 5%, provide significantly better correlation between drug sensitivity and infectious virus drug sensitivity at 5% wheat germ extract with little apparent correlation at 10% or 20%. The art has to date failed to recognize a connection between wheat germ extract concentration and drug sensitivity, recommending the use of high concentrations of wheat germ extract to maximize protein synthesis. Thus, the literature's teaching that wheat germ extract should be used at higher concentrations (e.g., 20% or higher) does not result in a cell-free system providing optimum sensitivity for drug screening, as observed at 5% wheat germ or lower, as provided by the present invention.

The present invention relates to a cell-free system for expressing proteins using 3 to 5 % wheat germ extract. Accordingly, the claimed subject matter provides compositions and methods useful for expressing proteins and for assaying modulators of viral proteins and capsid assembly.

The present invention provides a cell-free system for:
a. expressing a protein of interest, comprising components necessary for expression of the protein of interest, or
b. assembling a viral capsid, comprising components necessary for expression of at least one viral protein comprising the viral capsid, wherein the cell-free system further comprises 3 to 5% wheat germ extract. In an exemplary embodiment, the protein of interest is a viral protein. In various embodiments, the viral protein is a viral capsid protein. In a selected embodiment, the protein of interest is a capsid interacting protein. In a selected embodiment, the protein of interest is a host protein which undergoes assembly in a manner analogous to capsid proteins, i.e. most likely due to catalysis of formation of specific multi-protein complexes by other proteins in the cytoplasm.

In an exemplary embodiment, the protein is a non-viral protein that forms a multiprotein complex. In selected embodiments, the multiprotein complex participates in diseases comprising central nervous system disorders, metabolic disorders, oncologic disorders, or immunologic disorders. In an exemplary embodiment, the protein of interest is a bacterial or parasitic protein.

The viral protein can be from any virus or family of viruses. In an exemplary embodiment, the viral protein is from a virus which is a member of a viral family selected from the group consisting of *Flaviviridae, Togaviridae, Bunyaviridae, Arenaviridae, Filoviridae, Poxviridae, Orthomyxoviridae, Rhabdoviridae, Herpesviridae, Coronaviridae, Paramyxoviridae, Hepadnaviridae, Bornaviridae, Picornaviridae, Retroviridae, Reoviridae, Papillomaviridae, Adenoviridae, Astroviridae, Polyomaviridae.*

In addition to the 3 to 5% wheat germ extract, an exemplary composition of the invention further includes components necessary or useful to ensure that the expression system expresses the desired protein in the desired amount and/or form. In an exemplary embodiment, the further components of the composition include one or more of a buffer, an amino acid, and a nucleic acid transcript. In various embodiments the composition further comprises one or more of a detectable moiety, ATP, GTP, creatine phosphate, a labeled amino acid, myristoyl CoA lithium salt, an RNase inhibitor, creatine kinase, and a tRNA. In an exemplary embodiment, the labeled amino acid comprises [35S] methionine. In an exemplary embodiment, the nucleic acid transcript is derived from an *in vitro* transcription reaction. In an exemplary embodiment, the nucleic acid transcript encodes a viral protein, e.g., a viral capsid protein. In an exemplary embodiment, the nucleic acid transcript encodes a viral capsid interacting protein. In an embodiment, the buffer comprises a member selected from potassium acetate, spermine, and dithiothreitol or other reducing agents and a combination thereof.

In another embodiment, a method of expressing a protein of interest using a cell-free system as described herein is provided. In an exemplary embodiment, the protein of interest is a viral protein, e.g., a viral capsid protein. In an exemplary embodiment, the protein of interest is a viral capsid interacting protein.

In another embodiment, the invention provides a method of assembling a viral capsid using a cell-free system of the invention.

In an exemplary embodiment, the invention provides a method assembling non-viral proteins in a multiprotein complex. In selected embodiments, the methods assemble a multiprotein complex that participates in diseases comprising central nervous system disorders, metabolic disorders, oncologic disorders, or immunologic disorders. In an exemplary embodiment, the methods assemble a multiprotein complex of bacterial or parasitic proteins.

In various embodiments, there is provided a method of testing whether a compound modulates a cellular function. An exemplary method comprises introducing the compound to a cell-free system of the invention, and determining whether the cellular function is modulated.

The invention also provides a method of testing whether a compound modulates viral capsid assembly due to an effect on host proteins or on the viral capsid proteins themselves. The method includes introducing the compound to a cell-free system of the invention and determining whether the viral capsid (or other protein) assembly is modulated.

### BRIEF DESCRIPTION OF THE DRAWINGS

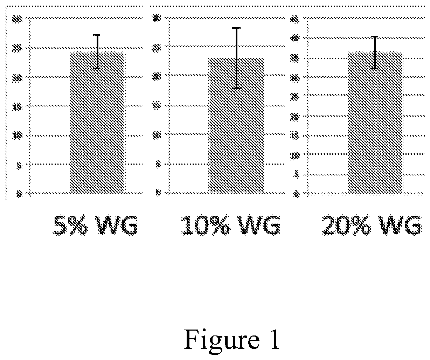

**Figure 1** illustrates quantitation of protein synthesis at a composition of 5%, 10%, or 20% wheat germ extract at 26 °C by western blotting 2 µL of translation extract in triplicates, and plotted as arbitrary density units upon quantitation of band intensity using an alkaline phosphatase conjugated secondary antibody-based colorimeteric method. Cell-free translation was performed in the WG cell-free protein synthesizing system, programmed with mRNA encoding Hepatitis C virus core polypeptide, at a composition of 5%, 10% or 20% wheat germ extract, in the absence (DMSO control), or presence of compounds whose efficacy had been assessed against infectious virus in cell culture and found to represent a gradation of potencies from weak to strong. A quantitation of protein synthesis at each percentage of wheat germ by western blotting of 2uL of translation extract (triplicates), plotted as arbitrary density units upon quantitation of band intensity using an alkaline phosphatase conjugated secondary antibody-based colorimeteric method. Conclusion: the level of protein synthesis is roughly the same at each percentage wheat germ, but slightly lower at 5% WG than either 10% or 20%.

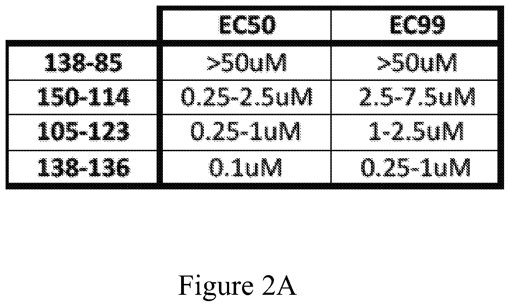

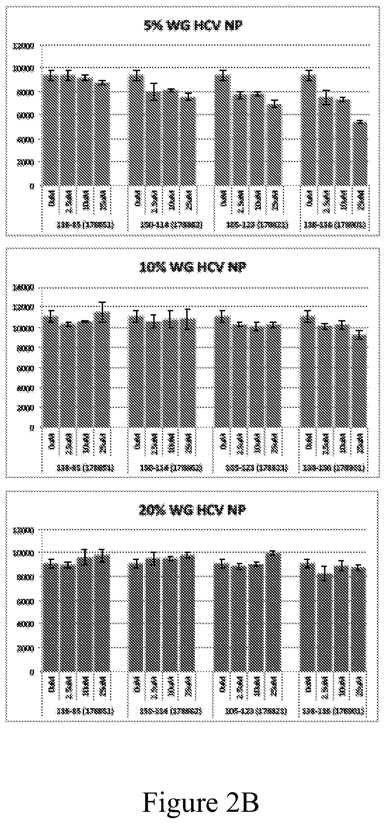

**Figure 2** illustrates the sensitivity and correlation between compound potency against Hepatitis C (HCV) synthesized at a composition of 5%, 10%, or 20% wheat germ extract in the absence (DMSO control) or presence of compounds. (A) Compound efficacy ranges from extremely weak (compound 138-85) to the most potent (compound 138-136). Conclusion: Compound potency against infectious virus in cell culture ranged from weak (EC50 > 50uM) to strong (EC50 = 100nM). (B) Correlation of drug potency on plate screen at each wheat germ percentage to drug potency against infectious virus. Conclusion: Drug sensitivity displays greatest correlation to infectious virus drug sensitivity at 5% wheat germ extract with little apparent correlation at 10 or 20%. Thus, the literature's teaching that wheat germ extract should be used at 20% or higher misses the point of optimum sensitivity for drug screening which is observed at 5% wheat germ or lower, as provided by the present invention.

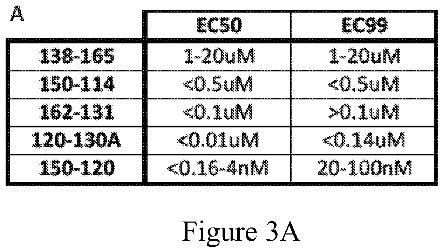

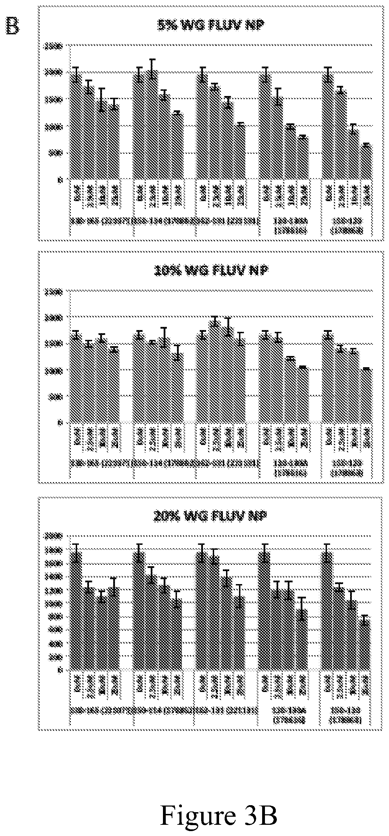

**Figure 3** illustrates the sensitivity and correlation between compound potency against influenza (FLUV) synthesized at a composition of 5%, 10%, or 20% wheat germ extract in the absence (DMSO control) or presence of compounds. (A) Compound efficacy ranges from extremely weak (compound 138-165) to the most potent (compound 150-120). (B) Correlation of drug potency using plate screen at each wheat germ percentage to drug potency against FLUV. As can be seen for FLUV, as for HCV in the previous figure, the sensitivity and correlation between compound potency against infectious virus and compound potency on the plate screen is greatest at 5% WG and is lost at 10% WG concentration, and reappears at 20% to some extent but not in full correlation with potency against infectious virus. Thus, the points made for HCV in Figure 1, apply to significant extent also to FLUV.

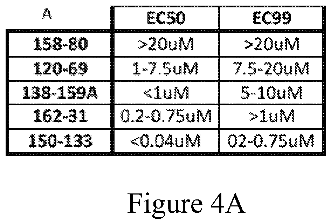

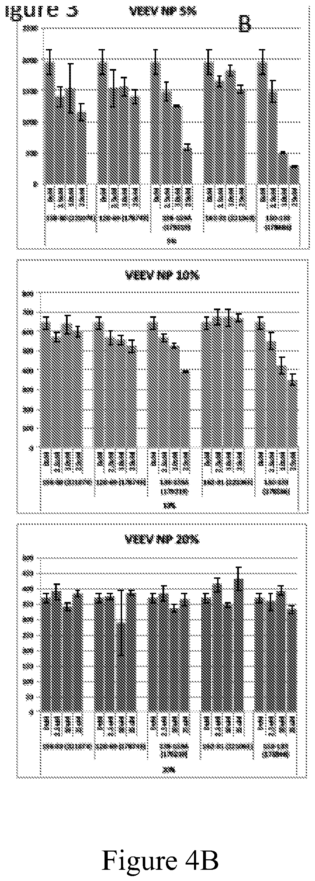

**Figure 4** illustrates the sensitivity and correlation between compound potency against Venezuelan Equine Encephalitis (VEEV) synthesized at a composition of 5%, 10%, or 20% wheat germ extract in the absence (DMSO control) or presence of compounds. (A) Compound efficacy ranges from extremely weak (compound 158-80) to the most potent (compound 150-133). (B) Correlation of drug potency using plate screen at each wheat germ percentage to drug potency against VEEV. As can be seen for VEEV, as for HCV in the previous figure, the sensitivity and correlation between compound potency against infectious virus and compound potency on the plate screen is greatest at 5% WG and is dampened at 10% and largely lost at 20% WG concentrations. Thus, the points made for HCV in Figure 1, apply also to VEEV.

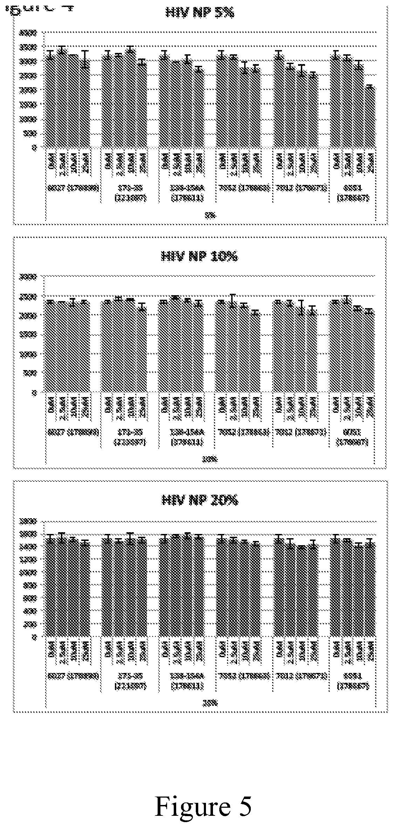

**Figure 5** illustrates the sensitivity and correlation between compound potency against Human Immunodeficiency Virus (HIV) synthesized at a composition of 5%, 10%, or 20% wheat germ extract in the absence (DMSO control) or presence of compounds. (A) Compound efficacy ranges from extremely weak (compound 6027) to the most potent (compound 6051). As can be seen for HIV, as for the previous three viral families represented in Figures 1-4, the sensitivity of the plate screen is greatest at 5% WG, at which concentration the greatest correlation to potency against infectious virus is seen. This sensitivity and correlation is dampened or lost at higher WG concentrations. Thus, in four of four cases, as demonstrated in figures 1-4, the cell-free system displays greatest drug sensitivity and best correlation to potency against infectious virus, at low WG concentrations.

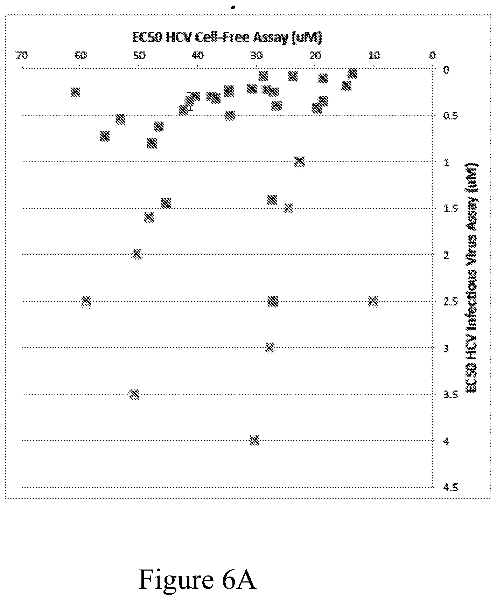

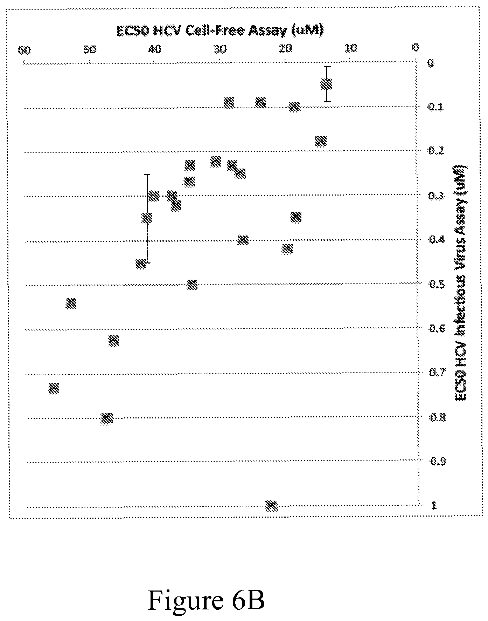

**Figure 6** illustrates the EC₅₀ observed in the HCV capsid protein-programmed cell-free system. (A) In order to establish the fidelity of the cell-free translation-based anti-capsid assembly drug screen, the EC₅₀ observed in the HCV capsid protein-programmed cell-free system was plotted versus the EC₅₀ against infectious HCV in cell culture. If activity in the cell- free system correlates with activity against infectious virus, the data points from structure-activity relationship optimization should project into the right upper quadrant, as observed. Furthermore, analogs that are more potent on other viral families are indicated in blue, off of the high correlation line. (B) Zoom of the most potent analogs from part (A) reveals more clearly the projection of analogs into the right upper quadrant.

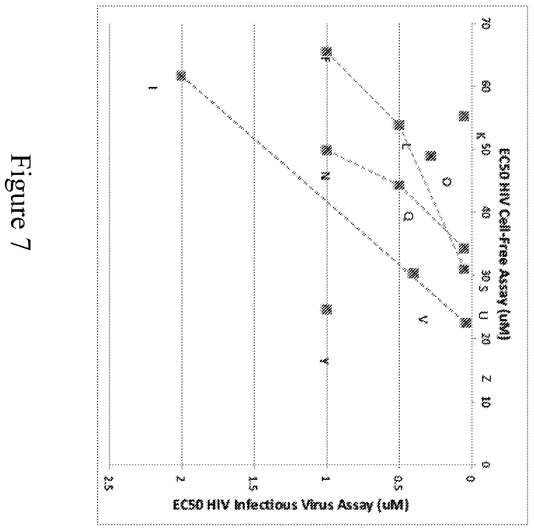

**Figure 7** illustrates the EC₅₀ observed in the HIV capsid protein-programmed cell-free system. Correlation of efficacy of analogs against HIV capsid assembly in the cell-free system (x-axis) versus against infectious virus in cell culture (y-axis) as was shown for HCV in figure 6. The parent compound is shown in red, with analogs depicted in purple. Structure-activity relationships are indicated by dotted lines connecting the related compounds that comprise a given sub pharmacophore.

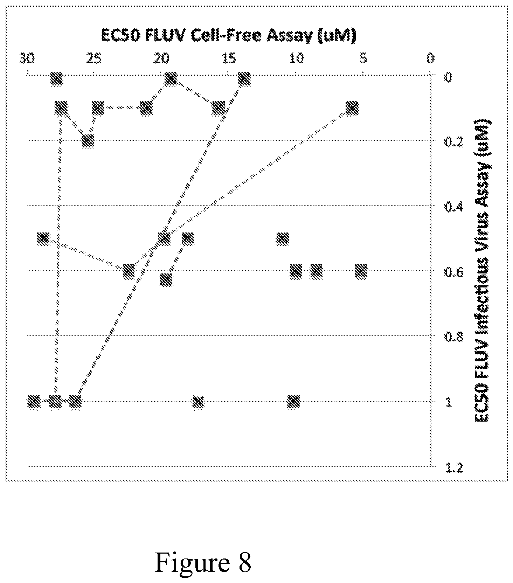

**Figure 8** illustrates the EC₅₀ observed in the FLUV capsid protein-programmed cell-free system. Correlation of efficacy of analogs against FLUV capsid assembly in the cell-free system (x-axis) versus against infectious virus in cell culture (y-axis) as was shown for HCV in figure 6 and HIV in figure 7. The parent compound is shown in red. Structure-activity relationship (SAR) is indicated by dotted lines connecting the related compounds that comprise a given sub-pharmacophore. As for HIV and FLUV, SAR project towards the right upper quadrant of the graph, as expected for good cell-free to authentic virus anti-viral compound correlation.

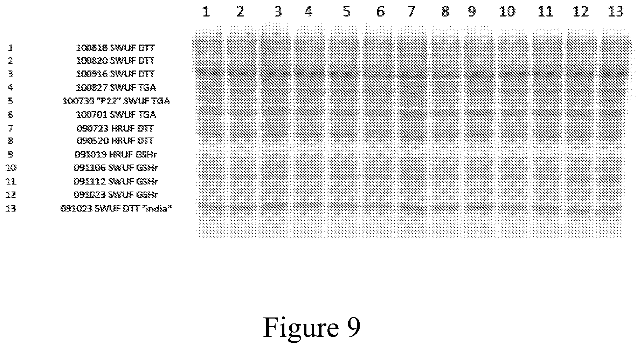

**Figure 9** illustrates the uniformity of 5 % wheat germ preparations among source and preparation conditions. Samples of 13 wheat germ extract preps were diluted 50 µL to 1 mL and 20 µL was taken and mixed with 20 µL of SDS gel sample buffer with 10% βME and heated at 100°C/5 min. 10 µL was loaded on SDS PAGE. The gel was fixed in 10% acetic acid and 50% methanol when the dye front reached the bottom and then stained with 0.1% Coomassie brilliant blue and destained to completion.

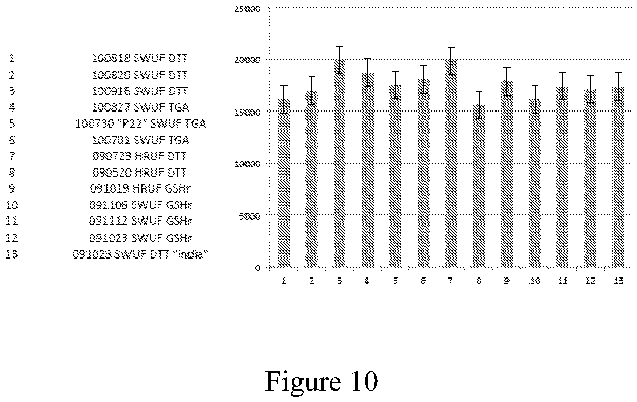

**Figure 10** illustrates the quantitation of scanned lanes from Figure 9 plotted as arbitrary integrated density units. This analysis quantifies the compositional similarity of different wheat germ preps and reveals the extracts to be highly reproducible in protein content as measured by Coomassie stainable protein.

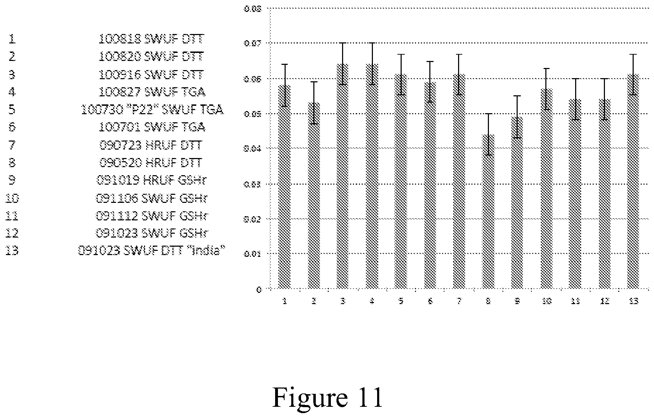

**Figure 11** illustrates the A260 absorbance spectrum of 5% wheat germ preps. The absorbance spectrum scan reveals a major A260 peak in the 5% wheat germ extract. This peak is used to define the range of absorbance values associated with each of the 13 5% wheat germ extract preps of Figure 9.

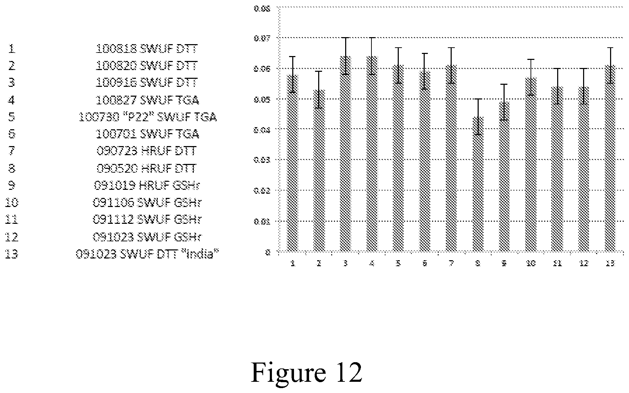

**Figure 12** illustrates the A280 absorbance spectrum of 13 5% wheat germ preps of Figure 9. The absorbance spectrum scan reveals no major A280 peaks in the 5% wheat germ extracts, due largely to the enormity of the A260 absorbance spectrum.

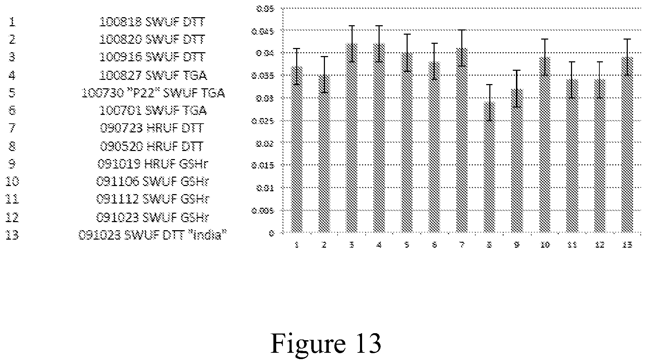

**Figure 13** illustrates the A260/A280 absorbance spectrum ratio. Because of the uniformity of A260 nm and A280 nm values from various wheat germ extract preparations, irrespective of source and a range of buffer conditions, the A260/A280 absorbance ratio was calculated as another measure of preparation uniformity.

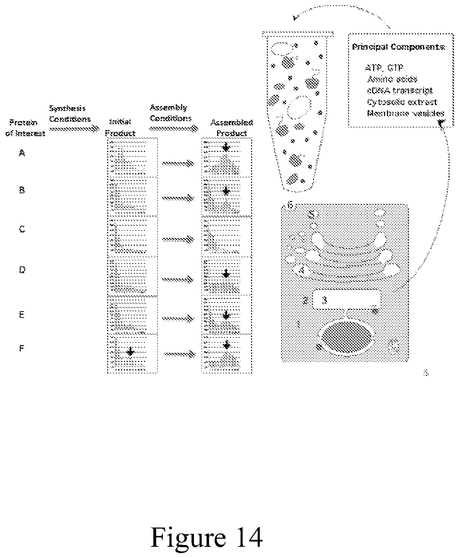

**Figure 14** illustrates cell-free systems for protein biogenesis applied to drug discovery for CNS disease. The biogenesis of six proteins (A-F) implicated in various CNS disorders were explored using the 5% wheat germ cell-free protein-synthesizing systems. The sucrose gradient profiles of initial and final products (to the right in blue), synthesis and assembly were dissociated in all cases. In all except C, conditions were established to successfully reconstitute formation of high molecular weight structures. For several putative CNS disorders, small molecule inhibitors were identified and validated in cells.

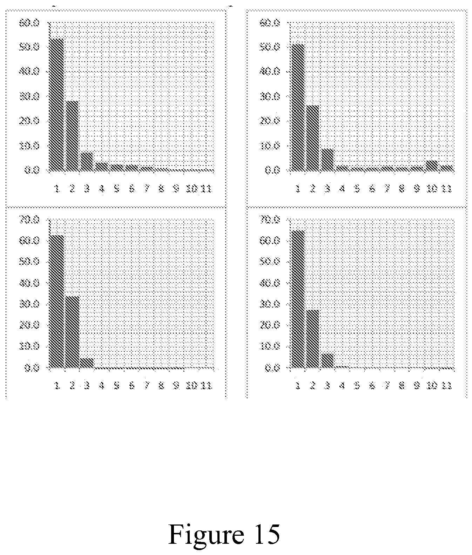

**Figure 15** illustrates sucrose gradient analysis of newly synthesized carbonic anhydrase (panels above) and alpha globin (panels below), two proteins whose mature oligomeric state does not involve large high molecular weight structures of >20S. Left panel is of gradient profile quantified from SDS PAGE and autoradiography after 35S labeled methionine synthesis at 26°C/1h, followed by treatment with puromycin to terminate protein synthesis and release chains. Right panel is of gradient profile quantified from SDS PAGE and autoradiography after subsequent incubation at 34°C/2h. Failure of conversion into high molecular weight structures of these newly synthesized proteins whose mature forms are limited to low molecular weight oligomeric forms suggests that assembly as observed for viral capsid proteins or known oligomeric non-viral proteins is not a form of non-specific aggregation, but rather represents substrate-selective assembly. Thus, this figure suggests that the present invention is selective for a subset of biologically relevant and medically important proteins, but not to the many proteins that do not form large multiprotein complexes upon cell-free translation and incubation under assembly conditions.

### DETAILED DESCRIPTION OF THE INVENTION

### Introduction

In various embodiments, the invention provides a cell-free system comprising 3 to 5% wheat germ extract and methods for expressing viral proteins using these systems. The cell-free systems of the invention are useful for assembling viral capsids. Also provided are methods for testing whether a compound modulates cellular function using a cell-free system of the invention.

Cell-free systems are generally useful for capsid assembly in that a variety of manipulations are possible. For example, reactions within the system can be separated into a protein synthesis phase and an assembly phase. This slows down the naturally occurring capsid assembly phase and allows manipulations to be performed to determine optimal modulation of viral protein synthesis and capsid assembly. One advantage of slowing down capsid assembly in a cell-free system is this allows for a correlation between the efficacy of known modulators of live viruses in cell culture and the efficacy of the same modulators in the cell-free system. Another advantage is that transient targets (e.g. host proteins that work catalytically to promote capsid formation) can be identified.

The present invention surprisingly demonstrates that those concentrations of wheat germ extraction, which are significantly lower than those long accepted in the art as standard and necessary concentration are very efficacious. It has long been accepted in the art that wheat germ extract should be used at concentrations above 20% in cell-free expression systems (See e.g., Erikson and Blobel, Methods Enz 96:38-50 (1983); and Lingappa and Thielen, Methods Mol Biol. 485: 185-95 (2009)). According to the compositions and methods of the present invention, capsid assembly is as robust at 5% wheat germ extract as it is at 20%. However, it is demonstrated herein that little or no correlation exists between antiviral efficacy against live viruses in cell culture and the ability to inhibit capsid assembly in cell-free systems using 20% wheat germ extract. What one wishes to achieve is the optimal balance between the level of synthesis and the amount of factors. Importantly, it is shown herein that efficacy of antiviral compounds in cell-free systems is most closely correlated to virus sensitivity in cell culture using wheat germ extract concentrations of 5%. At greater than 5%, drug sensitivity is diminished.

### Definitions

Unless otherwise noted, the technical terms used herein are according to conventional usage as understood by persons skilled in the art. Definitions of common terms in molecular biology may be found in standard texts (e.g. Benjamin Lewin, Genes V, published by Oxford University Press, 1994 (ISBN 0-19854287-9); Kendrew et al. (eds.), The Encyclopedia of Molecular Biology, published by Blackwell Science Ltd, 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8)).

The phrase "cell-free system," as used herein, refers to any type of system capable of synthesizing proteins in vitro in the absence of viable cells. An exemplary system is a cell-free protein synthesis system derived from wheat germ extract.

The term "expressing" and "expression," as used herein, refer to the production of a protein, peptide, or nucleotide sequence, and include transcription into an RNA product, post-transcriptional modification and/or translation into a protein product or polypeptide from a DNA encoding that product, as well as possible post-translational modifications.

The terms "polypeptide" or "peptide" or "protein" are used interchangeably herein, to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Macromolecular structures such as polypeptide structures can be described in terms of various levels of organization. For a general discussion of this organization (*see,* e.g., Alberts et al., Molecular Biology of the Cell (3rd ed., 1994) and Cantor and Schimmel, Biophysical Chemistry Part I: The Conformation of Biological Macromolecules (1980)). "Primary structure" refers to the amino acid sequence of a particular peptide. "Secondary structure" refers to locally ordered, three dimensional structures within a polypeptide. These structures are commonly known as domains, e.g., enzymatic domains, extracellular domains, transmembrane domains, pore domains, and cytoplasmic tail domains. Domains are portions of a polypeptide that form a compact unit of the polypeptide and are typically 15 to 350 amino acids long. Exemplary domains include domains with enzymatic or other functional activity. Typical domains are made up of sections of lesser organization such as stretches of 3-sheet and a-helices. "Tertiary structure" refers to the complete three dimensional structure of a polypeptide monomer. "Quaternary structure" refers to the three dimensional structure formed by the noncovalent association of independent tertiary units.

A "protein of interest," "desired polypeptide," "desired protein," or "target protein," as used herein, are interchangeable and refer to a whole protein molecule, including but not limited to, viral capsid proteins, or a portion thereof, i.e., cytoplasmic domain or other domain of a protein.

The term "virus" or "viral," as used herein, refers to minute infectious agents, which, with certain exceptions, are not observable by light microscopy, lack independent metabolism, and are able to replicate only within a living host cell. Some exceptions include, but are not limited to, the cell-free translation system described herein. These individual particles (i.e., for example, virions) typically comprise nucleic acid and a protein shell or coat; some virions also have a lipid containing membrane. The term virus encompasses all types of viruses, including animal, plant, phage, and other viruses.

The term "viral capsid" or "capsid," as used herein, refers to the protein coat that surrounds the viral nucleic acid. Viral capsids have interior surfaces and exterior surfaces. The interior surface of a viral capsid is the surface that is normally exposed to the viral nucleic acid. The exterior surface of a viral capsid is the surface that is generally exposed to the environment. The phrase "viral capsid assembly" refers to the process of arranging viral capsid proteins in a manner sufficient to generate a viral capsid.

The term "capsid interacting protein," as used herein, refers to protein that interacts with a viral capsid either during or after its assembly. The capsid interacting protein may be endogenous to a virus, may be exogenously added, or present with the cell-free extract. Capsid interacting proteins can include, but are not limited to, capsid chaperones and proteins that have catalytic actions favoring capsid formation.

The term "components," as used herein, refers to constituents of the cell-free system necessary to incorporate a naturally occurring or non-natural amino acid into a growing polypeptide chain. For example, components can include, but are not limited to, buffers, amino acids, nucleic acid transcripts, ATP, GTP, creatine phosphate, labeled amino acids, myristoyl CoA lithium salts, RNase inhibitors, creatine kinases, and tRNAs. Components are described in greater detail herein.

The phrase "detectable moiety" or "conjugate" refers to any atom, molecule or a portion thereof, the presence, absence or level of which is directly or indirectly monitorable. A variety of detectable moieties are well known to those skilled in the art, and can be any material detectable by spectroscopic, photochemical, biochemical, immunochemical, electrical, optical or chemical means. Such detectable labels can include, but are not limited to, magnetic beads, fluorescent dyes, radiolabels, enzymes, and colorimetric labels such as colloidal gold or colored glass or plastic beads.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code. Amino acids can be referred to herein by either their commonly known three letter symbols or by the one-letter symbols recommended by the IUPAC-IUB Biochemical Nomenclature Commission. Nucleotides, likewise, can be referred to by their commonly accepted single-letter codes. Amino acid substitutions, deletions or additions to individual or a small percentage of amino acids in the encoded sequence is a conservatively modified variant, where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

The term "nucleic acid" refers to deoxyribonucleotides or ribonucleotides and polymers thereof in either single- or double-stranded form, and complements thereof. Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions can be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). The term nucleic acid is used interchangeably with gene, cDNA, mRNA, oligonucleotide, and polynucleotide. A particular nucleotide sequence also implicitly encompasses "splice variants," which as the name suggests, are products of alternative splicing of a gene. After transcription, an initial nucleic acid transcript can be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Any products of a splicing reaction, including recombinant forms of the splice products, are included in this definition.

The phrase *"in vitro* transcription reaction," as used herein, refers to a transcription reaction that takes place in a cell-free environment using largely purified components, for example, purified DNA template and purified DNA-dependent RNA polymerase.

The term "modulates" or "modulated," as used herein, means to interact with a target either directly or indirectly so as to alter the activity of the target, including, for example, to inhibit the activity of the target, or to limit or reduce the activity of the target. Accordingly, the phrase "modulates a cellular function" means to alter the function of a way, which can include, but is not limited to, inhibition of protein synthesis or inhibition of protein assembly into molecular structures such as viral capsids.

The term "test compound" or "compound" or "drug candidate" or "modulator" or grammatical equivalents, as used herein, describes any molecule, either naturally occurring or synthetic, e.g., protein, oligopeptide (e.g., from about 5 to about 25 amino acids in length, preferably from about 10 to 20 or 12 to 18 amino acids in length, preferably 12, 15, or 18 amino acids in length), small organic molecule, polysaccharide, lipid, fatty acid, polynucleotide, oligonucleotide, etc., to be tested for the capacity to directly or indirectly modulation tumor cell proliferation. The test compound can be in the form of a library of test compounds, such as a combinatorial or randomized library that provides a sufficient range of diversity. Test compounds are optionally linked to a fusion partner, e.g., targeting compounds, rescue compounds, dimerization compounds, stabilizing compounds, addressable compounds, and other functional moieties. Conventionally, new chemical entities with useful properties are generated by identifying a test compound (called a "lead compound") with some desirable property or activity, e.g., inhibiting activity, creating variants of the lead compound, and evaluating the property and activity of those variant compounds. Often, high throughput screening (HTS) methods are employed for such an analysis. Compounds can be inhibitors, activators, or modulators of, for example, nucleic acid and polypeptide sequences, and are used to refer to activating, inhibitory, or modulating molecules identified using *in vitro* and in vivo assays of the nucleic acid and polypeptide sequences. Inhibitors are compounds that, e.g., bind to, partially or totally block activity, decrease, prevent, delay activation, inactivate, desensitize, or down regulate activity or expression of, for example, nucleic acids or polypeptides derived from the cell-free system described herein, e.g., antagonists. Activators are compounds that increase, open, activate, facilitate, enhance activation, sensitize, agonize, or up regulate, for example, nucleic acids or polypeptides derived from the cell-free system described herein, e.g., agonists. Inhibitors, activators, or modulators also include genetically modified versions of the proteins derived from the cell-free system, e.g., versions with altered activity, as well as naturally occurring and synthetic ligands, substrates, antagonists, agonists, antibodies, peptides, cyclic peptides, nucleic acids, antisense molecules, ribozymes, or small chemical molecules, for example.

The phrase "small organic molecule" refers to an organic molecule, either naturally occurring or synthetic, that has a molecular weight of from about 50 to about 2500 daltons, preferably less than about 2000 daltons, preferably between about 100 and about 1000 daltons, more preferably between about 200 and about 500 daltons.

The term "biopharmaceutical" or "biopharmaceutical compound," as used herein, refers to any compound such as a protein, peptide, polypeptide, antibody, or the like, which can be expressed endogenously in a biological system under genetic control and which confers biological activity toward pharmaceutical or therapeutic use. The biopharmaceutical or biopharmaceutical compound can be constitutively or inducibly expressed. The biological system can be an in vivo biological system and/or an *in vitro* biological system.

### Cell-free systems

Cell-free extracts of wheat germ provide cytosolic factors critical for translation, and support the *in vitro* translation of a wide variety of mRNAs into protein. The translation mechanism is sufficiently conserved so that a cell-free system can translate both prokaryotic and eukaryotic mRNAs with high efficiency. The optimal concentration of wheat germ extract can be determined for each RNA sequence to be translated.

In an exemplary embodiment, the invention provides a cell-free system (composition) for expressing a protein of interest, including viral proteins, comprising 3 to 5% wheat germ extract. In various embodiments, the invention provides a cell-free system for viral capsid assembly comprising 3 to 5% wheat germ extract.

The method for producing the wheat germ extract used in the present invention is not particularly limited, as long as the wheat germ extract enables the cell-free protein synthesis when template nucleic acid, amino acids, and an energy source and the like are supplied. In an exemplary process, an extract obtained by separating wheat germ from albumen in a wheat seed, followed by extraction and purification from the wheat germ, is used as a wheat germ extract. One skilled in the art recognizes that wheat germ extract can be prepared from a wheat seed (See, e.g., Erikson and Blobel, Methods Enz 96:38-50 (1983); PNAS 97:559-564 (2000)). One skilled in the art also recognizes that wheat germ can be commercially purchased (e.g. Promega, American Biosciences, etc).

Cell-free translation systems using wheat germ extract can utilize a wide variety of components in addition to the wheat germ extract for efficient translation. Described herein are basic components useful for efficient translation of viral proteins using wheat germ extract. However, one skilled in the art recognizes that additional components might be useful for translation and/or capsid assembly depending on the desired properties of the proteins and/or capsids produced the desired production conditions and other variables. The choice of the proper components for a cell-free system of the invention is well within the capabilities of those of skill in the art. The use of wheat germ extract for capsid assembly is also known in the art, and is described in more detail, for example, in Lingappa and Thielen, Methods Mol Biol. 485: 185-95 (2009), and US Pat. No. 7,638,269.

In an exemplary embodiment, the composition of the invention further includes a buffer. Cell-free translation systems generally require an appropriate compensating buffer. Potassium and magnesium concentrations of the wheat germ translation system can have dramatic effects on the efficiency of translation, and the compensating buffer is used to adjust the ion concentration of the total translation reaction to an optimum that can be determined for each mRNA being translated. Buffers can include further components for efficient protein expression, which include, but are not limited to, potassium acetate, amines (e.g., spermine), and sulfur compounds (e.g., dithiothreitol).

The composition of the invention also optionally includes a nucleic acid encoding a protein or a portion thereof. In an exemplary embodiment, the translation mixture contains transcript nucleic acid or a fragment thereof that encodes one or more viral protein. In this embodiment, the cell-free translation system involves two linked reactions: *in vitro* transcription and cell-free translation. RNA can be obtained by any method known in the art including, but not limited to, isolating mRNA or by making *in vitro* RNA transcripts from DNA cloned into a vector containing an RNA polymerase promoter. RNA molecules can also be generated in the same reaction vessel used for the translation reaction. In an exemplary embodiment, the RNA is generated in situ by addition of, for example, SP6 polymerase to the reaction mixture along with the viral protein coding region or cDNA.

In an exemplary embodiment, a sample containing a virus of interest or a bodily fluid of an individual infected with a virus of interest, or infected cells from an individual, is used a source of viral nucleic acid encoding the protein for the virus. This can then be engineered behind an appropriate promoter (e.g. for SP6 polymerase), amplified by PCR and purified for transcription-linked translation. The fluid may be any bodily fluid including, without limitation, blood, serum, plasma, lymphatic fluid, urine, sputum, cerebrospinal fluid, and the like.

The endogenous mRNA present in the wheat germ extract can compete with the exogenous RNA for ribosomes and factors required for translation. It is therefore optionally advantageous to reduce the concentration of endogenous RNA by treating the prepared extract with nuclease. Such nucleases are well known in the art, and can include, but are not limited to, micrococcal nuclease from *Staphylococcus aureus.*

Methods known in the art are used to maintain energy levels sufficient to maintain protein synthesis. In an exemplary embodiment, additional nucleotide energy sources are added during the reaction. In various embodiments, energy is maintained by addition of an energy source such as creatine phosphate/creatine phosphokinase. In an exemplary embodiment, ATP and GTP concentrations present in a standard translation mixture known in the art are sufficient to support both protein synthesis and capsid formation.

In an exemplary embodiment, the virus has a myristolated intermediary. For such viruses, one can add sufficient myristoyl coenzyme A (MCoA) with or without acceptable salts to the system to enable capsid assembly. The concentration required may vary according to the particular experimental conditions, and can therefore be determined empirically.

In various embodiments, the cell-free translation systems of the invention comprise further components including, but not limited to, RNase inhibitors, ribonuclease inhibitors, protease inhibitors, microsomal membranes, and tRNAs, either alone or in combination.

### Viral protein expression and capsid assembly

The present invention also provides a method of expressing a protein using a composition of the invention. In an exemplary embodiment, the cell- free system is used to mimic capsid biogenesis and assembly. In the cell-free system, viral capsid transcripts are translated in the presence of wheat germ extract that contains soluble factors necessary for capsid protein translation and subsequent capsid assembly (Lingappa et al. J. Cell Biol 136:567-581 (1997)). Both cytosolic and membrane proteins present in the wheat germ extract may be involved in capsid assembly and/or viral replication. Integral membrane proteins can include transmembrane proteins. In those embodiments utilizing viruses requiring membrane proteins for capsid assembly, appropriate membranes can be added to the cell-free translation mixture. It is further possible to supplement the cell-free translation mixture with other exogenous proteins, such as chaperone proteins that can for example, facilitate the assembly of capsid intermediates. Assembly of capsids in the cell-free system minimally requires expression of only the particular viral protein(s) that are involved in capsid assembly. Once expressed, polypeptides proceed to assemble into capsids that are catalyzed by host factors.

After incubation for a time sufficient to produce capsids, products of the cell-free reaction can be analyzed to determine sedimentation value, buoyant density, and electron microscopy appearance. Together these form a sensitive set of measurements for integrity of capsid formation.

Synthesized viral proteins can be detected in any manner known in the art. In an exemplary embodiment, radiolabeled capsid polypeptides using labeled amino acids are used. In one embodiment using this approach, ³⁵S methionine is added to the translation mixture. Following *in vitro* expression, velocity sedimentation gradients can generate fractions that are aliquoted into loading buffers and run on a standard SDS-PAGE gel. The gel can be exposed to film that generates autoradiographs showing the amount of ³⁵S labeled viral protein in different fractions of the velocity sedimentation gradients. Alternatively, as is known in the art, a phosphoimager can be used to visualize radiolabeled viral proteins.

In various embodiments, antibodies, e.g., commercially available antibodies, are used to detect successful protein expression or capsid assembly. Alternatively, antibodies can be specifically raised against proteins of interest, as is well known in the art.

Viral protein expression and capsid assembly in the cell-free system of the invention is useful for viruses from any family including, without limitation, *Flaviviridae, Togaviridae, Bunyaviridae, Arenaviridae, Filoviridae, Poxviridae, Orthomyxoviridae, Rhabdoviridae, Herpesviridae, Coronaviridae, Paramyxoviridae, Hepadnaviridae, Bornaviridae, Picornaviridae, Retroviridae, Reoviridae, Papillomaviridae, Adenoviridae, Astroviridae, Polyomaviridae.*

### Non-viral proteins

The present invention also provides a method of expressing non-viral proteins using a composition of the invention. In an exemplary embodiment, the cell-free system is used to mimic a pathway of assembly into a multi-protein complex for which the viral capsid serves as an analogous model. Figure 14 illustrates the use of 5% wheat-germ extract to dissect various CNS-related proteins, including the DISCI gene into a synthesis phase and an assembly phase. Because host proteins involved in viral capsid assembly must exist in the host for other purposes, the non-viral proteins that are the substrates for those endogenous assembly pathways should be equally effectively usable for drug screening by the present invention. Thus, the present invention should be applicable not only to CNS disease, but also to proteins involved in other disorders including but not limited to metabolic, oncologic, and immunologic diseases. The present invention can be further used to mimic a bacterial or parasitic protein that forms a multiprotein complex that when disrupted, ameliorates bacterial or parasitic disease. What is required for applicability of the present invention is that the newly synthesized proteins in question share the ability to use other proteins in the extract to assist or facilitate their assembly into distinct multiprotein complexes.

### Assays for modulators

In some embodiments, modulation of proteins, including viral proteins, can be assessed by determining the effect of a compound on expression, folding, and assembly of the protein in the cell-free system of the invention. In other embodiments, modulation of viral capsid assembly can be assessed by determining the effect of a compound on capsid assembly using a cell-free system of the invention. In some embodiments, modulation of capsid interacting proteins, including but not limited to capsid assembly chaperone proteins, can be assessed by determining the effect of a compound on expression of the protein in the cell-free system of the invention. Modulation can further include, but is not limited to, modulation of infection, replication, receptor binding, cell entry, particle formation, and the like.

An advantage of using the cell-free system of the present invention is that the process of capsid formation is slowed down, thus allowing for the targeting of capsid assembly processes for modulators of capsid assembly. An advantage of using cell-free systems of the invention, is an increased sensitivity for detecting compounds that otherwise would not be detected at higher wheat germ concentrations.

Measurement of modulation of a viral protein and/or viral capsid assembly can be performed using a variety of assays, *in vitro, in vivo,* and *ex vivo.* The assays described herein can use a full length viral protein, a variant, a mutant or a fragment thereof. A suitable physical, chemical or phenotypic change that affects activity, e.g., enzymatic activity, cell surface marker expression, viral replication and proliferation can be used to assess the influence of a test compound on the proteins expressed. The assay can also make use of one or more drug designed to block or alter protein activity, capsid assembly, or the associations of chaperones with viral proteins. The assay can also identify modulators of viral capsid assembly intermediates. Moreover, genomic nucleic acid can also be encapsidated into capsids, which can be used to design drugs that interfere with encapsidation and with the design of assay systems that examine the mechanism of action of drugs that inhibit encapsidation.

The binding assay can be either solid state or soluble. The protein or viral capsid can be bound to a solid support, either covalently or non-covalently. Often, the *in vitro* assays of the invention are substrate or ligand binding or affinity assays, either non-competitive or competitive. Other *in vitro* assays include measuring changes in spectroscopic (e.g., fluorescence, absorbance, refractive index), hydrodynamic (e.g., shape), chromatographic, or solubility properties for the protein.

A high throughput binding assay can be performed in which the viral protein is contacted with a potential modulator and incubated for a suitable amount of time. The potential modulator can be bound to a solid support, and the protein added. Alternatively, the protein is bound to a solid support. A wide variety of modulators can be used, including, but not limited to, small organic molecule, or a biopharmaceutical or biological entity, such as a protein, e.g., an antibody or peptide, a sugar, a nucleic acid, e.g., an antisense oligonucleotide or a ribozyme or siRNA, or a lipid. A wide variety of assays can be used to identify viral protein-modulator binding, including labeled protein-protein binding assays, electrophoretic mobility shifts, immunoassays, enzymatic assays, and the like. In some cases, the binding of the candidate modulator is determined through the use of competitive binding assays, where interference with binding of a known ligand or substrate is measured in the presence of a potential modulator. Either the modulator, the known ligand, or substrate is bound first; then the competitor is added. After the protein is washed, interference with binding, either of the potential modulator or of the known ligand or substrate, is determined. Often, either the potential modulator or the known ligand or substrate is labeled.

In high throughput assays, either soluble or solid state, it is possible to screen up to several thousand different modulators or ligands in a single day. In particular, each well of a microtiter plate can be used to run a separate assay against a selected potential modulator, or, if concentration or incubation time effects are to be observed, every 5-10 wells can test a single modulator. Thus, a single standard microtiter plate can assay about 350 (e.g., 384) modulators. If 1536 well plates are used, then a single plate can easily assay from about 100-about 1500 different compounds. It is possible to assay many plates per day; assay screens for up to about 6,000, 20,000, 50,000, or more than 100,000 different compounds are possible using integrated systems.

High throughput screening methods involve providing a combinatorial small organic molecule or peptide library containing a large number of potential therapeutic compounds (potential modulator or ligand compounds) can be used. Such "combinatorial chemical libraries" or "ligand libraries" are then screened in one or more assays to identify those library members (particular chemical species or subclasses) that display a desired characteristic activity. The compounds thus identified can serve as conventional "lead compounds" or can themselves be used as potential or actual therapeutics.

A combinatorial chemical library is a collection of diverse chemical compounds generated by either chemical synthesis or biological synthesis, by combining a number of chemical "building blocks" such as reagents. For example, a linear combinatorial chemical library such as a polypeptide library is formed by combining a set of chemical building blocks (amino acids) in every possible way for a given compound length (i.e., the number of amino acids in a polypeptide compound). Millions of chemical compounds can be synthesized through such combinatorial mixing of chemical building blocks.

Preparation and screening of combinatorial chemical libraries is well known to those of skill in the art. Such combinatorial chemical libraries include, but are not limited to, peptide libraries (see, e.g., U.S. Patent 5,010,175, Furka, Int. J. Pept. Prot. Res. 37:487-493 (1991) and Houghton et al., Nature 354:84-88 (1991)). Other chemistries for generating chemical diversity libraries can also be used. Such chemistries include, but are not limited to: peptoids (e.g., PCT Publication No. WO 91/19735), encoded peptides (e.g., PCT Publication No. WO 93/20242), random bio-oligomers (e.g., PCT Publication No. WO 92/00091), benzodiazepines (e.g., U.S. Pat. No. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J Amer. Chem. Soc. 114:6568 (1992)), nonpeptidal peptidomimetics with glucose scaffolding (Hirschmann et al., J Amer. Chem. Soc. 114:9217-9218 (1992)), analogous organic syntheses of small compound libraries (Chen et al., I Amer. Chem. Soc. 116:2661 (1994)), oligocarbamates (Cho et al., Science 261 : 1303 (1993)), and/or peptidyl phosphonates (Campbell et al., J Org. Chem. 59:658 (1994)), nucleic acid libraries (see Ausubel, Berger and Sambrook, *supra*), peptide nucleic acid libraries (see, e.g., U.S. Patent 5,539,083), antibody libraries (see, e.g., Vaughn et al., Nature Biotechnology, 14(3):309-314 (1996) and PCT/US96/10287), carbohydrate libraries (see, e.g., Liang et al., Science, 274: 1520-1522 (1996) and U.S. Patent 5,593,853), small organic molecule libraries (see, e.g., benzodiazepines, Baum C&EN, Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337; benzodiazepines, 5,288,514, and the like).

### Mammalian extracts

The concepts illustrated by the present invention are equally applicable to non-wheat germ extracts capable of supporting both protein synthesis and protein assembly. Thus, the lowest concentration of mammalian protein extracts capable of de novo synthesis of proteins is likely to be the most effective system for multiprotein assembly, by analogy to the present invention. One skilled in the art would be capable of using the information provided here for wheat germ extracts and applying it to development of extracts from mammalian cells.

The following examples are intended to illustrate exemplary embodiments of the invention and do not limit the scope of the invention.

### EXAMPLE 1

Viral capsid assembly was examined in cell-free protein synthesizing systems having a composition of 5%, 10%, or 20% wheat germ extract in the presence of compounds (or DMSO) control whose efficacy had been assessed against infectious viruses in cell culture.

### Results

Protein synthesis was first quantified at a composition of 5%, 10%, or 20% wheat germ extract by blotting 2µL of translation extract (triplicates), and plotted as arbitrary density units upon quantitation of band intensity using an alkaline phosphatase conjugated secondary antibody-based colorimeteric method using the Hepatitis C virus core polypeptide. Protein synthesis was roughly the same at each percentage of wheat germ extract **(****Figure 1****).**
Addition, protein synthesis was uniform regardless of wheat germ extract source or preparation conditions **(****Figures 9-13****),** and selective for a subset of biologically relevant and medically important proteins that form large multiprotein complexes **(****Figure 15****).**

Compounds that were shown to be potent against Hepatitis C, Influenza, Venezuelan Equine Encephalitis, and Human Immunodeficiency viruses in cell culture were then correlated for potency against respective viral capsid proteins using the cell-free protein synthesis system at different wheat germ extract concentrations.

### Hepatitis C (HCV)

Optimal wheat germ extract concentration was determined for compounds shown to be efficacious against Hepatitis C. Compound potency against infectious virus in cell culture ranged from weak (138-85; EC₅₀> 50µM) to strong (138-136; EC₅₀= 100nM) **(****Figure 2A** **and** **6****).** Drug sensitivity displayed a greatest correlation to infectious virus drug sensitivity at 5% wheat germ extract with little apparent correlation at 10 or 20% **(****Figure 2B****).**

### Influenza (FLUV)

Optimal wheat germ extract concentration was determined for compounds shown to be efficacious against Influenza. Compound potency against infectious virus in cell culture ranged from weak (138-65; EC₅₀ 1-20µM) to strong (150-120; EC₅₀< 0.16-4nM) **(****Figure 3A** **and** **Figure 8****).** Drug sensitivity displayed a greatest correlation to infectious virus drug sensitivity at 5% wheat germ extract. The correlation is lost at 10% wheat germ extract, and reappears to some extent at 20%, but not in full correlation with the potency against the infectious virus cell culture assay. **(****Figure 3B****).**

### Venezuelan Equine Encephalitis Virus (VEEV)

Optimal wheat germ extract concentration was determined for compounds shown to be efficacious against Venezuelan Equine Encephalitis Virus. Compound potency against infectious virus in cell culture ranged from weak (158-80; EC₅₀ > 20µM) to strong (150-133; EC₅₀< 0.4µM) **(****Figure 4A****).** Drug sensitivity displayed a greatest correlation to infectious virus drug sensitivity at 5% wheat germ extract and is greatly reduced at 10% wheat germ extract and mostly lost at 20% wheat germ extract. **(****Figure 4B****).**

### Human Immunodeficiency Virus (HIV)

Optimal wheat germ extract concentration was determined for compounds shown to be efficacious against Human Immunodeficiency Virus. Compound potency against infectious virus in cell culture is shown in **Figure 7****.** Drug sensitivity displayed a greatest correlation to infectious virus drug sensitivity at 5% wheat germ extract and is greatly reduced or lost at higher wheat germ extract concentrations **(****Figure 5****).**

### Methods

Cloned cDNAs encoding the protein of interest were engineered behind an SP6 bacteriophage promoter and mammalian 5' untranslated coding region, and PCR products were generated, gel purified, phenol/chloroform extracted, ethanol precipitated, and dissolved in sterile water and adjusted to 1 mg/mL. The products were sued to generate transcripts.

A transcription reaction is set up containing the following components at the indicated final concentration: Tris pH 7.9 45mM, MgAc 6mM, Spermidine 2mM, dithiothreitol 10mM, and 480µM each ATP, GTP, UTP, CTP, 500 units/mL SP6 polymerase, 25 units/mL of Rnasin, and 20 µg/mL of PCR product were incubated at 40°C for 2 1/2 hours, aliquoted, and frozen in liquid N2 and stored at -80°C.

The translation reaction is carried out in a 20µL volume in a 384 well plate format, with 1/5 of the volume comprising the transcription reaction. The balance of the reaction including the following components at the indicated concentration in the final translation reaction: ATP and GTP ImM each, creatine phosphate 4mM, each of the 20 amino acids used for protein synthesis at 40µM, Rnasin 4 µg/mL, creatine kinase 50 µg/mL, wheat germ extract with the A260/280 absorbance values indicated in **Figures 11-13****,** comprising approximately 5% or less of the translation volume.

## Claims

1. A cell-free system for:
a. expressing a protein of interest, comprising components necessary for expression of the protein of interest, or
b. assembling a viral capsid, comprising components necessary for expression of at least one viral protein comprising the viral capsid,
wherein the cell-free system further comprises 3 to 5% wheat germ extract.

2. A method of expressing a protein of interest, wherein the protein of interest is expressed in the cell-free system of claim 1, part a.

3. A method of assembling a viral capsid using the cell-free system of claim 1, part b.

4. A method of testing whether a compound modulates a cellular function, the method comprising
a. introducing the compound to the cell-free system of claim 1, part a; and
b. determining whether the cellular function is modulated, optionally wherein the compound is selected from the group consisting of a small organic molecule and a biopharmaceutical.

5. A method of testing whether a compound modulates viral capsid assembly, the method comprising:
a. introducing the compound to the cell-free system of claim 1, part b; and
b. determining whether the viral capsid assembly is modulated, optionally wherein the compound is selected from the group consisting of a small organic molecule and a biopharmaceutical.

6. The cell-free system of claim 1 or the method of any one of claims 2 or 3, wherein the protein of interest is:
i. a viral protein, for example a viral capsid protein;
ii. a capsid interacting protein;
iii. non-viral protein that forms a multiprotein complex, for example a bacterial or parasitic protein.

7. The cell-free system of claim 1, wherein the components comprise a member selected from the group consisting of a buffer, an amino acid, a nucleic acid transcript and a combination thereof and optionally further comprise a member selected from the group consisting of a detectable moiety, ATP, GTP, creatine phosphate, a labeled amino acid, for example [35S] methionine, myristoyl CoA lithium salt, an RNase inhibitor, creatine kinase, a tRNA and a combination thereof.

8. The cell-free system of claim 7 when dependent on claim 1, part a, wherein the nucleic acid transcript:
i) is assembled in an in vitro transcription reaction;
ii) encodes a viral protein, for example a viral capsid protein; or
iii) encodes a viral capsid interacting protein.

9. The cell-free system of claim 7 when dependent on claim 1, part b, wherein the nucleic acid transcript:
i) is assembled in an in vitro transcription reaction; or
ii) encodes proteins that comprise a viral capsid.

10. The cell-free system of claim 7, wherein the buffer comprises a member selected from the group consisting of potassium acetate, spermine, dithiothreitol and a combination thereof.

11. The cell-free system of claim 1, part b or claim 6, wherein the viral protein or viral capsid is from a viral family selected from *Flaviviridae, Togaviridae, Bunyaviridae, Arenaviridae, Filoviridae, Poxviridae, Orthomyxoviridae, Rhabdoviridae, Herpesviridae, Coronaviridae, Paramyxoviridae, Hepadnaviridae, Bornaviridae, Picornaviridae, Retroviridae, Reoviridae, Papillomaviridae, Adenoviridae, Astroviridae, Polyomaviridae, Anelloviridae, Parvoviridae, or Caliciviridae.*

## Patentansprüche

1. Zellfreies System zum:
a) Exprimieren eines relevanten Proteins, das die erforderlichen Bestandteile für die Expression des relevanten Proteins umfasst; oder
b) Assemblieren eines viralen Kapsids, das die erforderlichen Bestandteile für die Expression mindestens eines viralen Proteins umfasst, welches das virale Kapsid umfasst,
wobei das zellfreie System ferner 3 bis 5% Weizenkeimextrakt umfasst.

2. Verfahren zum Exprimieren eines viralen Kapsids unter Verwendung zellfreien Systems nach Anspruch 1 gemäß Teil a).

3. Verfahren zum Assemblieren eines viralen Kapsids unter Verwendung des zellfreien Systems nach Anspruch 1 gemäß Teil b).

4. Verfahren zum Testen, ob eine Verbindung eine zelluläre Funktion moduliert, wobei das Verfahren folgendes umfasst:
a) Einführen der Verbindung in das zellfreie System nach Anspruch 1 gemäß Teil a); und
b) Bestimmen, ob die zelluläre Funktion moduliert ist, wobei die Verbindung optional ausgewählt ist aus der Gruppe bestehend aus einem kleinen organischen Molekül und einem Biopharmazeutikum.

5. Verfahren zum Testen, ob eine Verbindung die Assemblierung von viralem Kapsid moduliert, wobei das Verfahren folgendes umfasst:
a) Einführen der Verbindung in das zellfreie System nach Anspruch 1 gemäß Teil b); und
b) Bestimmen, ob die Assemblierung von viralem Kapsid moduliert ist, wobei die Verbindung optional ausgewählt ist aus der Gruppe bestehend aus einem kleinen organischen Molekül und einem Biopharmazeutikum.

6. Zellfreies System nach Anspruch 1 oder Verfahren nach Anspruch 2 oder 3, wobei das relevante Protein folgendes ist:
i) ein virales Protein, wie etwa ein virales Kapsidprotein;
ii) ein Protein mit Kapsidinteraktion;
iii) nicht-virales Protein, das einen Multiproteinkomplex bildet, wie etwa ein bakterielles oder parasitäres Protein.

7. Zellfreies System nach Anspruch 1, wobei die Bestandteile ein Element umfassen, das ausgewählt ist aus der Gruppe bestehend aus einem Puffer, einer Aminosäure, einem Nukleinsäuretranskript und einer Kombination davon, und wobei sie optional ferner ein Element umfassen, das ausgewählt ist aus der Gruppe bestehend aus einem nachweisbaren Anteil, ATP, GTP, Kreatinphosphat, einer markierten Aminosäure, z.B. [35S] Methionin, Myristoyl-CoA-Lithiumsalz, einem RNase-Hemmer, Kreatinkinase, einer tRNA und einer Kombination davon.

8. Zellfreies System nach Anspruch 7 in Abhängigkeit von Anspruch 1, Teil a), wobei das Nukleinsäuretranskript:
i) in einer In-vitro-Transkriptionsreaktion assembliert ist;
ii) für ein virales Protein codiert, wie etwa ein virales Kapsidprotein; oder
iii) für ein virales Protein mit Kapsidinteraktion codiert.

9. Zellfreies System nach Anspruch 7 in Abhängigkeit von Anspruch 1, Teil b), wobei das Nukleinsäuretranskript:
i) in einer In-vitro-Transkriptionsreaktion assembliert ist; oder
ii) für Proteine codiert, die ein virales Kapsid umfassen.

10. Zellfreies System nach Anspruch 7, wobei der Puffer ein Element umfasst, das ausgewählt ist aus der Gruppe bestehend aus Kaliumacetat, Spermin, Dithiothreitol und einer Kombination davon.

11. Zellfreies System nach Anspruch 1, Teil b), oder Anspruch 6, wobei das virale Protein oder das virale Kapsid von einer viralen Familie stammt, ausgewählt aus *Flaviviridae, Togaviridae, Bunyaviridae, Arenaviridae, Filoviridae, Poxviridae, Orthomyxoviridae, Rhabdoviridae, Herpesviridae, Coronaviridae, Paramyxoviridae, Hepadnaviridae, Bornaviridae, Picornaviridae, Retroviridae, Reoviridae, Papillomaviridae, Adenoviridae, Astroviridae, Polyomaviridae, Anelloviridae, Parvoviridae oder Caliciviridae.*

## Revendications

1. Système acellulaire pour :
a. exprimer une protéine d'intérêt, comprenant des composants nécessaires à l'expression de la protéine d'intérêt, ou
b. assembler une capside virale, comprenant des composants nécessaires à l'expression d'au moins une protéine virale comprenant la capside virale,
le système acellulaire comprenant en outre de 3 à 5 % d'extrait de germe de blé.

2. Procédé d'expression d'une protéine d'intérêt, la protéine d'intérêt étant exprimée dans le système acellulaire selon la revendication 1, partie a.

3. Procédé d'assemblage d'une capside virale utilisant le système acellulaire selon la revendication 1, partie b.

4. Procédé pour tester si un composé module une fonction cellulaire, le procédé comprenant les étapes consistant à
a. introduire le composé dans le système acellulaire selon la revendication 1, partie a ; et
b. déterminer si la fonction cellulaire est modulée, éventuellement le composé étant choisi dans le groupe constitué par une petite molécule organique et un produit biopharmaceutique.

5. Procédé pour tester si un composé module un assemblage de capside virale, le procédé comprenant les étapes consistant à :
a. introduire le composé dans le système acellulaire selon la revendication 1, partie b ; et
b. déterminer si l'assemblage de capside virale est modulé, éventuellement le composé étant choisi dans le groupe constitué d'une petite molécule organique et d'un produit biopharmaceutique.

6. Système acellulaire selon la revendication 1 ou procédé selon la revendication 2 ou 3, la protéine d'intérêt étant :
i. une protéine virale, par exemple une protéine de capside virale ;
ii. une protéine interagissant avec la capside ;
iii. une protéine non virale qui forme un complexe multiprotéique, par exemple une protéine bactérienne ou parasitaire.

7. Système acellulaire selon la revendication 1, les composants comprenant un élément choisi dans le groupe constitué par un tampon, un acide aminé, un transcrit d'acide nucléique et une combinaison de ceux-ci et comprenant éventuellement en outre un élément choisi dans le groupe constitué par un fragment détectable, de l'ATP, de la GTP, une créatine phosphate, un acide aminé marqué, par exemple méthionine[35S], le sel de lithium CoA myristoyl, un inhibiteur RNase, une créatine kinase, un tRNA et une combinaison de ceux-ci.

8. Système acellulaire selon la revendication 7 lorsqu'elle dépend de la revendication 1, partie a, le transcript d'acide nucléique :
i) étant assemblé dans une réaction de transcription in vitro ;
ii) codant une protéine virale, par exemple une protéine de capside virale ; ou
iii) codant une protéine interagissant avec la capside virale.

9. Système acellulaire selon la revendication 7 lorsqu'elle dépend de la revendication 1, partie b, le transcript d'acide nucléique :
i) étant assemblé dans une réaction de transcription in vitro ; ou
ii) codant les protéines qui comprennent une capside virale.

10. Système acellulaire selon la revendication 7, le tampon comprenant un élément choisi dans le groupe constitué par l'acétate de potassium, la spermine, le dithiothréitol et une combinaison de ceux-ci.

11. Système acellulaire selon la revendication 1, partie b ou la revendication 6, la protéine virale ou la capside virale appartenatant à une famille virale choisie parmi *Flaviviridae, Togaviridae, Bunyaviridae, Arenaviridae, Filoviridae, Poxviridae, Orthomyxoviridae, Rhabdoviridae, Herpesviridae, Coronaviridae, Paramyxoviridae, Hepadnaviridae, Bornaviridae, Picornaviridae, Retroviridae, Reoviridae, Papillomaviridae, Adenoviridae, Astroviridae, Polyomaviridae, Anelloviridae, Parvoviridae* ou *Caliciviridae.*
